**Europäisches Patentamt**

(19) **European Patent Office**

**Office européen des brevets**

(11) Publication number: **0 134 647**
**A1**

(12) # EUROPEAN PATENT APPLICATION

(21) Application number: **84304513.9**

(51) Int. Cl.⁴: **C 13 K 1/02**

(22) Date of filing: **02.07.84**

(30) Priority: **07.07.83 US 511782**
**07.07.83 US 511783**

(43) Date of publication of application:
**20.03.85 Bulletin 85/12**

(84) Designated Contracting States:
**AT BE CH DE FR GB IT LI LU NL SE**

(71) Applicant: **BIOMASS INTERNATIONAL INC.**
**P.O. Box 1031 2743 Midland Drive**
**Ogden Utah 84402(US)**

(72) Inventor: **Neves, Alan M.**
**1596 24th Street**
**Ogden Utah 84401(US)**

(72) Inventor: **Stokes, Barry O.**
**1591 East 1220 North**
**Logan Utah 84321(US)**

(72) Inventor: **Reynolds, James H.**
**589 South Angel**
**Layton Utah 84041(US)**

(74) Representative: **Wharton, Peter Robert et al,**
**P.R. Wharton & Co. Beckett's Bank Chambers 19**
**Cheapside**
**Bradford West Yorkshire BD1 4HR(GB)**

(54) **Method and apparatus for continuously hydrolyzing cellulosic material.**

(57) The present invention is directed to methods and apparatus for continuously hydrolyzing cellulosic material. Cellulosic material is continuously introduced into the top end of a reaction vessel while an acid solution is continuously introduced into the bottom end thereof so as to contact the cellulosic material in a countercurrent fashion. The acid hydrolyzes the cellulosic material to sugars, leaving lignin residue. The sugars and lignin are continuously removed from the reaction vessel at opposing ends. The density of the cellulosic material within the reaction vessel is controlled such that the density is within the range of about 10 to about 30 pounds per cubic foot. The liquid volume within the reaction vessel is reduced by displacing a portion of the liquid within the reaction vessel with a vapor. The novel apparatus and methods maximize sugar recovery, while maintaining high concentration of sugar in the recovered sugar solution and while minimizing degradation of the sugar to waste products.

./...

Fig. 1

METHOD AND APPARATUS FOR CONTINUOUSLY

HYDROLYZING CELLULOSIC MATERIAL

_____

Background

1.    The Field of the Invention

The present invention relates to methods and apparatus for hydrolyzing cellulosic material. In particular, the present invention is directed to countercurrent flow methods and apparatus for continuously hydrolyzing cellulosic material in order to produce sugars which are capable of fermentation into alcohols.

2.    The Prior Art

Many different processes have been developed for hydrolyzing cellulosic material with acid to recover sugars. The sugars produced may be used for a variety of purposes, for example, as a fermentation substrate in the production of alcohol, as an animal feedstock, or as a starting material for making molasses. Prior art processes for hydrolyzing cellulosic material with acid to produce sugars include: (1) simple batch processes, (2) percolation batch processes, (3) cocurrent continuous processes, and (4) percolation continuous processes.

Although the chemical structure and makeup of various cellulosic materials somewhat differ, cellulosic materials are comprised primarily of lignin, lignin-bound crystalline cellulose, and hemicellulose. While the hemicellulose can be readily separated and hydrolyzed by treatment with a weak acid, the remaining lignin-bound cellulose requires much more rigorous treatment for separation and hydrolysis.

The hydrolysis products of the hemicellulose are primarily in the form of pentose sugars (e.g., xylose, arabinose, and ribose) and hexose sugars (e.g., mannose and galactose). On the other hand, hydrolysis of the crystalline cellulose yields primarily glucose with the lignin remaining unhydrolyzed in solid form.

Significant problems have been encountered in the prior art processes for hydrolyzing cellulosic material with acid to recover sugars. One major problem is the fact that as soon as the hemicellulose and cellulose in the cellulosic material are hydrolyzed to sugars, the acid continues to act on the sugars so as to degrade them into undesirable waste products. Generally, pentose sugars are first degraded to furfural and then to formic acid and polymeric condensation products; hexose sugars are first degraded to hydroxymethyl furfural and then to levulinic acid, formic acid, and polymeric condensation products.

The rate of the acid hydrolysis reaction proceeds according to equation (1) below:

0134647

$$\frac{a}{a_o} = e^{-k_1 t} \qquad (1)$$

In equation (1), t represents the amount of time elapsed, $a_o$ represents the amount of cellulose in the cellulosic material present at time 0, a represents the amount of cellulose remaining at time t, $k_1$ is the first order rate constant for the cellulose hydrolysis reaction, and e is the base of the system of natural logarithms having the approximate numerical value of 2.71828.

Once sugar is formed by the hydrolysis reaction, it is immediately subject to degradation; the rate of degradation of the sugar follows equation (2) below:

$$\frac{b}{b_o} = e^{-k_2 t} \qquad (2)$$

In equation (2), t represents the amount of time elapsed, $b_o$ represents the amount of sugar present at time 0, b represents the amount of sugar remaining at time t, $k_2$ represents the first order rate constant for the sugar degradation reaction, and e is the base of the system of natural logarithms having the approximate numerical value of 2.71828.

The hydrolysis and degradation rate constants, $k_1$ and $k_2$, respectively, are fixed for any given temperature and acid concentration. With an increase in temperature or acid concentration, the values of $k_1$ and $k_2$ are also increased. Importantly, however, the value of the hydrolysis rate constant $k_1$ increases significantly more rapidly than the value of the degradation rate constant $k_2$ as the temperature or acid concentration is increased; thus, hydrolysis is favored at high $k_1$ values.

In the typical simple batch processes of the prior art, a quantity of cellulosic material is placed into a vessel and a quantity of hot acid is introduced into the vessel. The acid is allowed to hydrolyze the cellulosic material to sugars, the contents of the vessel are cooled to stop the reaction, and the sugar solution is separated and recovered from the remaining lignin.

In light of the competing rates of the hydrolysis and degradation reactions discussed above, the best recovery of the potentially available sugar in the cellulosic feedstock material within the vessel of the simple batch processes of the prior art has been limited to about 50-55%. Practical recoveries of industrial processes have usually been significantly lower. This is because in simple batch processes, the sugars produced remain in the vessel for the same length of time as the cellulosic feedstock material. Thus, while it is necessary to allow the hot acid to act on the cellulosic material for a substantial period of time so as to maximize the amount of cellulosic material hydrolyzed by the acid, much of the sugars produced during this time are degraded, thereby resulting in poor total recovery.

In the typical percolation batch processes of the prior art, a quantity of cellulosic material is placed into a vessel, and a hot acid solution is percolated through the vessel to remove the sugars as they are formed. Although continuous removal of the sugars as they are formed reduces degradation of the sugars, the sugar concentration in the continuously flowing acid solution is relatively low.

Thus, in the prior art percolation batch processes, a higher percentage of the total sugars recovered is achieved at the expense of the relatively low concentration of the sugar solution produced. Low sugar concentrations are extremely undesirable because much time and substantial costs are involved in concentrating dilute sugar solutions for subsequent uses, e.g., where the sugar solutions are to be used as a substrate in fermentation processes to produce alcohols such as ethanol or as a starting material for making molasses.

In the typical cocurrent continuous processes of the prior art, a quantity of cellulosic material and a quantity of hot acid are mixed together in a continuously flowing stream. The reaction is stopped after a suitable period of time by cooling the mixture or by neutralizing the acid, and the sugar solution produced is separated from the remaining lignin solids. Unfortunately, the cocurrent continuous processes suffer from substantially the same problems as the simple batch processes of the prior art. Since the residence time of the acid and sugar solution in the reaction vessel is the same as the residence time for the cellulosic solids in these processes, the maximum recovery of the potentially available sugar in the cellulosic material using these processes has generally been no better than in the simple batch processes.

In the typical percolation continuous processes of the prior art, a quantity of cellulosic material is placed into a vessel and a quantity of hot acid is introduced radially into the vessel in a continuous fashion. The prior art percolation continuous processes also suffer from substantially the same problems as the prior art percolation batch processes. Since the residence time of the solid cellulosic material is substantially higher than the residence time of the acid solution in such continuous flow systems, higher recovery of the total sugars available in the cellulosic material is achieved by removing the sugars as they are produced. However, such processes unfortunately result in relatively low sugar concentrations in the continuously flowing acid solution.

In an attempt to achieve high total sugar recoveries and still maintain an acceptable sugar concentration in the sugar solution produced, a countercurrent hydrolysis process such as that disclosed in U.S. Patent No. 2,681,871 to L.C. Wallace was developed. In the Wallace countercurrent hydrolysis process, cellulosic material and acid are continuously and countercurrently introduced into a vessel so as to contact each other. The sugar solution produced is continuously withdrawn from one end of the vessel, while the remaining lignin solids are withdrawn from the other end thereof. The continuous countercurrent process of Wallace reduces the amount of time the sugars produced within the vessel are exposed to the acid, thereby decreasing the extent of sugar degradation. Moreover, the continuous addition of cellulosic material into the vessel provided for higher sugar concentrations in the sugar solution produced than was achieved in other prior art processes.

Thus, the continuous countercurrent hydrolysis process developed by L.C. Wallace solved some of the problems encountered in the prior art processes. Nevertheless, many critical parameters (such as the percent of available sugar recovered and the sugar concentration of the recovered sugar solution) have not been maximized in such prior art countercurrent processes.

Obviously, it would be desirable to develop a process for hydrolyzing cellulosic material wherein a maximum amount of the available sugar within the cellulosic material is

0134647

recovered. Moreover, it would be highly desirable to maintain a high concentration of sugar in the sugar solution recovered in a process for hydrolyzing cellulosic material.

Thus, it will be appreciated that what is needed in the art is a method and apparatus for hydrolyzing cellulosic material wherein the advantages of a continuous countercurrent process are maintained while optimizing the important parameters, such as the percent of available sugar recovered and the sugar concentration of the recovered sugar solution. Such an apparatus and method are disclosed and claimed herein.

## Brief Summary and Objects of the Invention

The present invention relates to a countercurrent flow method and apparatus for continuously hydrolyzing cellulosic material which method and apparatus are characterized by maximal sugar recovery and concentration of sugar in the recovered sugar solution.

Cellulosic material is continuously introduced into a top end of a reaction vessel, while a hot acid solution is continuously introduced into a bottom end of the reaction vessel so as to contact the cellulosic material in a countercurrent fashion. The counterflowing acid solution acts to hydrolyze the cellulose (including hemicellulose) in the cellulosic material to produce a sugar solution and

-8-

lignin. The sugar solution is continuously removed from the top end of the reaction vessel, and the lignin is continuously removed from the bottom end thereof.

The cellulosic material forms a bed within the reaction vessel, and the density of the bed of cellulosic material is controlled in the range from about 10 to about 30 pounds per cubic foot. In one preferred embodiment, the density of the bed is controlled by reducing the particle size of the cellulosic material prior to introduction of the cellulosic material into the reaction vessel. In another preferred embodiment, the density of the bed of cellulosic material is controlled by mechanically compacting the cellulosic material which forms the bed within the reaction vessel. By increasing the density of the bed of cellulosic material, the residence time of the sugar solution within the reaction vessel is decreased, as discussed hereinafter.

The residence time of the sugar solution produced within the reaction vessel may be further minimized by vapor displacement of the liquid volume contained within the reaction vessel. Such vapor displacement may be achieved by sparging gas into the reaction vessel or by flashing off a portion of the hot acid solution within the reaction vessel. Moreover, vapor displacement of the liquid volume within the reaction vessel may be done in lieu of, or in tandem with, controlling the density of the bed of cellulosic material to maximize the sugar recovery in the process.

Thus, in the apparatus and method of the present invention, the residence time of the sugar solution formed within the reaction vessel is decreased either by increasing the density of the bed of cellulosic material within the reaction vessel, or by vapor displacement of the liquid volume within the reaction vessel, or both. By decreasing the residence time of the sugar solution within the reaction vessel, the extent of the exposure of the sugar solution to the acid is correspondingly minimized, thereby minimizing degradation of the sugar products.

Moreover, maintaining a high bed density further results in the requirement for less reactor space, thereby enabling smaller reaction vessels to be employed and further enhancing the ability of the apparatus to maintain minimal residence times for the sugar solution produced within the reaction vessel.

It is, therefore, an object of the present invention to provide an improved countercurrent flow apparatus and method for continuously hydrolyzing cellulosic material.

Another object of the present invention is to provide an apparatus and method for continuously hydrolyzing cellulosic material wherein a maximum amount of the available sugar content of the cellulosic material is recovered, while maintaining a high concentration of sugar in the sugar solution recovered from the process.

A further object of the present invention is to provide an apparatus and method for continuously hydrolyzing cellulosic material wherein the required size for the reaction vessel of the method and apparatus is significantly smaller than those used in the prior art processes.

Still another object of the present invention is to provide an apparatus and method for continuously hydrolyzing cellulosic material wherein degradation of the sugars produced to undesirable waste products is minimized.

These and other objects and features of the present invention will become more fully apparent from the following description and appended claims taken in conjunction with the accompanying drawings.

Brief Description of the Drawings

Figure 1 is a partial cross-sectional view of a presently preferred embodiment. of the apparatus and method of the present invention for continuously hydrolyzing cellulosic material.

Figure 2 is a top plan view of the restriction grate of the preferred embodiment of Figure 1.

<u>Detailed Description of the Preferred Embodiments</u>

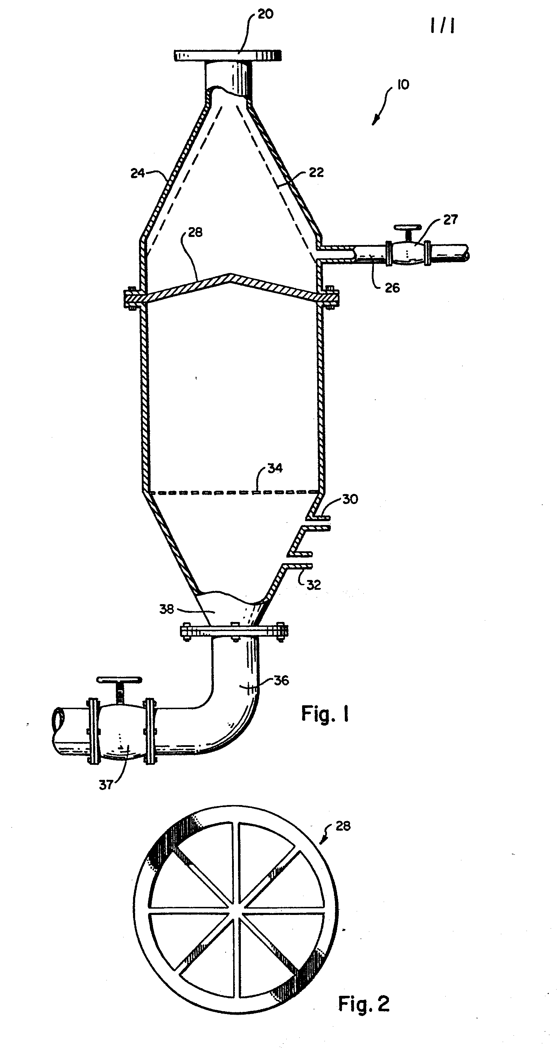

Reference is now made to the drawings wherein like parts are designated with like numerals throughout. One preferred embodiment of the apparatus for continuously hydrolyzing cellulosic material is generally designated 10 in the illustration of Figure 1.

As shown in Figure 1, apparatus 10 is provided with a feed inlet 20 for receiving cellulosic material into the apparatus. Inlet 20 is in communication with a conventional high pressure feeding device (not shown), such as an extruder, a tapered screw feed, a ram feeder, a lock hopper, a compression auger, combinations of the foregoing, or any other suitable high pressure feeding device. The high pressure feeding device provides the necessary motive force for moving cellulosic material through a reaction vessel 24.

Feed inlet 20 thus provides for the pressurized introduction of a cellulosic feedstock into the reaction vessel 24. Mounted within vessel 24 are two restriction grates 28 and 34. Restriction grates 28 and 34 are preferably configured as shown in Figures 1 and 2. As will be discussed in more detail hereinafter, restriction grates 28 and 34 serve to compact the cellulosic material within reaction vessel 24.

Vessel 24 is further provided with an inlet 30 for injecting acid solution into the vessel and preferably with an outlet 32 for withdrawing acid solution from the vessel 24 and recirculating the acid solution back into vessel 24 via inlet 30.

An outlet 38 is formed at the bottom end of reaction vessel 24 to allow for the removal of lignin from the vessel 24, and an outlet conduit 36 receives the lignin from outlet 38. A pulse valve 37 is provided in outlet conduit 36 to permit control of the flow of the lignin therethrough.

Another outlet 26 is disposed at the top end of reaction vessel 24 to allow for the removal of sugar solution from vessel 24. A valve 27 is provided in outlet 26 to permit control of the flow of the sugar solution therethrough. A conically-shaped screen 22 is preferably placed within apparatus 10 to filter the sugar solution before exiting outlet 26.

The operation of the preferred embodiment illustrated in Figure 1 and one preferred embodiment of the method for continuously hydrolyzing cellulosic material are described below. Referring to Figure 1, the cellulosic starting material to be treated (e.g. corn residue, forestry, feed lot, crop, urban, or industrial wastes) is continuously introduced into apparatus 10 through inlet 20 and into reaction vessel 24.

As the cellulosic material enters reaction vessel 24, a bed of cellulosic material (not shown) is formed within

-13-

reaction vessel 24. Upon reaching restriction grate 28, the flow of the cellulosic material is substantially retarded, thereby causing the cellulosic material to become compacted. Restriction grate 34 further retards the flow of the cellulosic material, resulting in further compaction of the cellulosic material.

It will be appreciated that any number of restriction grates may be used to achieve the desired amount of compaction of the cellulosic material in accordance with the present invention. Moreover, the upper restriction grate 28 which serves to compact the cellulosic material could be used alone, without restriction grate 34, if desired. However, in the presently preferred embodiment of Figures 1 and 2, two restriction grates are employed. In this presently preferred embodiment, lower restriction grate 34 not only serves to provide further compaction of the cellulosic bed of material, but grate 34 also provides a lower boundary for the cellulosic bed and serves to promote an even flow of acid up through the cellulosic bed.

It will also be appreciated that configurations other than those shown in Figures 1 and 2 for restriction grates 28 and 34 are possible. In choosing a suitable design for each of grates 28 and 34, factors such as the amount of compaction desired and promotion of an even flow of acid through the cellulosic bed should be considered.

An acid solution is injected into the reaction vessel 24 through inlet 30 so as to flow upwardly towards outlet 26. Although sulfuric acid ($H_2SO_4$) is the presently preferred acid used in the present invention, it will be recognized that other suitable acids are well-known in the art for achieving hydrolysis of cellulosic material and may thus also be employed. For example, such acids as hydrochloric acid (HCl), hydrofluoric acid (HF), and phosphoric acid ($H_3PO_4$) may also be used.

The upwardly flowing acid solution meets the downwardly flowing cellulosic material in a countercurrent fashion. Upon contact between the cellulosic material and acid solution, the cellulosic material is hydrolyzed to form a sugar solution, with lignin remaining as the waste product. The sugar solution is carried with the acid solution upwardly through vessel 24 where it exits the vessel at outlet 26. Screen 22 acts to filter the exiting sugar solution and to provide a barrier for the bed of cellulosic material within vessel 24.

If further agitation of the acid solution within reaction vessel 24 is desired, a portion of the acid solution may be withdrawn from outlet 32 and recycled for subsequent reinjection into vessel 24 through inlet 30. Such recycling of the acid solution serves to provide a turbulence zone in the region where the lignin exits vessel 24. Such a turbulence zone is helpful in achieving a uniform flow of acid upwards through the bed of cellulosic material.

Upon hydrolysis of the cellulosic material, the lignin waste product is forced through restriction grate 34 by the continued action of the incoming cellulosic material into vessel 24. The lignin is then suspended in the turbulent mixing zone and exits vessel 24 through outlet 38 into outlet conduit 36. The flow of lignin through outlet conduit 36 may be controlled through the periodic opening and closing of pulse valve 37.

The pressure within vessel 24 is allowed to build to a predetermined point, and then the pressure is released by opening pulse valve 37. This release in pressure serves to shock or jar the bed of cellulosic material within vessel 24, and thus helps to prevent undesirable clogging or obstruction of the vessel by the cellulosic material. After such a pressure release, pulse valve 37 is again closed and the pressure is again allowed to build within vessel 24. The periodic opening and closing of pulse valve 37 may be adjusted as desired.

If desired, the acid remaining with the lignin may be separated from the lignin and recycled to acid inlet 30. Additionally, if desired, the lignin may be washed and dried for subsequent use as fuel or chemical feedstock.

Using the novel apparatus and method of the present invention, maximum recovery of the available sugar in the cellulosic material is achieved, and in addition, the concentration of sugar in the recovered sugar solution is maintained at a relatively high level, and the amount of sugars degraded to waste products is minimized. As will be explained in more detail hereinbelow, such results are obtained in the present invention through compaction of the bed of cellulosic material and/or by minimizing the residence time of the liquid (and therefore the residence time of the sugar solution) within reaction vessel 24.

The extent of the recovery of potential available sugar in the cellulosic starting material is dependent upon the residence time of the solid cellulosic material within the reaction vessel. A solids residence time of about seven half lives of the hydrolysis reaction is sufficient to convert nearly 100% of the cellulosic starting material into hydrolyzed sugar products. Of course, the half life of the hydrolysis reaction will depend on various reaction conditions such as temperature and acid concentration. The solids residence time may be defined as follows:

$$\text{Solids Residence Time} = \frac{\text{Total Solids in Reaction Vessel}}{\text{Solids Flow Rate}}$$

$$= \frac{\text{Volume of Reaction Vessel} \times \text{Density of Solids}}{\text{Solids Flow Rate}} \qquad (3)$$

The concentration of sugar in the sugar solution which exits the reaction vessel depends primarily on the flow ratio of the solid cellulosic material to the liquid acid solution. High solid/liquid flow ratios yield high sugar concentrations, while conversely, low solid/liquid flow ratios correspondingly produce low sugar concentrations in the resulting sugar solution.

A competing factor to achieving a high concentration of sugar in the sugar solution is that of sugar degradation. The residence time of the sugar solution within the reaction vessel determines the amount of degradation of the sugars within the vessel. By decreasing the residence time of the sugar solution within the vessel, the amount of degradation is correspondingly decreased. Thus, in order to maximize the sugar concentration of the recovered sugar solution while minimizing degradation of the sugar solution, the solids/liquids flow ratio is maintained at an appropriately high level, and the residence time of the sugar solution in the reaction vessel is minimized by decreasing the effective volume of the liquid sugar solution in the vessel.

The residence time of the liquid (containing the sugar solution) within the reaction vessel is defined as follows:

$$\text{Liquid Residence Time} = \frac{\text{Liquid Volume in Reaction Vessel}}{\text{Liquid Flow Rate}} \quad (4)$$

In order to maintain a preselected sugar concentration in the sugar solution produced, the liquid flow rate must be kept at a constant value which is determined by the selected solids flow rate and the sugar recovery rate. The sugar concentration may thus be defined as:

$$\text{Sugar Concentration} = \frac{\text{Weight of Sugar}}{\text{Weight of Sugar} + \text{Weight of Liquid}} \quad (5)$$

Where:

$$\text{Weight of Sugar} = \begin{array}{l} \text{Weight of Solids} \times \text{Fraction} \\ \text{of Sugar in Solids} \times \text{Rate of} \\ \text{Recovery of Sugars} \times \text{Time} \end{array} \quad (6)$$

With the liquid flow rate thus fixed, the liquid residence time can only be decreased in accordance with equation (4) by decreasing the effective liquid volume within the reaction vessel. The present invention achieves a reduction in the liquid volume of the reaction vessel by compacting the solid cellulosic material within the vessel and/or by vapor displacement of the liquid volume within the reaction vessel.

By compacting the cellulosic material within the reaction vessel, the required volume of the reaction vessel is reduced for a given solids residence time (see Equation (3) above), thereby reducing the volume of sugar solution within the reaction vessel at any given time. Thus, compacting the bed of cellulosic material within the reaction vessel has the effect of reducing the residence time of the sugar solution within the vessel, thereby minimizing degradation of the sugars and thereby reducing the required size of reaction vessel.

Compaction of the cellulosic material in accordance with the apparatus and method of the present invention can be accomplished in several ways. As discussed previously, restriction grates 28 and 34 provide for compaction of the cellulosic material by restricting the flow of the cellulosic material.

In another preferred embodiment, compaction of the cellulosic material is accomplished by reducing the particle size of the cellulosic material prior to its introduction into apparatus 10. Particle reduction causes a substantial pressure drop across the cellulosic bed such that the bed is compacted by the counter flowing acid solution. In this embodiment, the cellulosic material is first pressurized with steam to a first pressure within the range of about 250 pounds per square inch (psi) to about 1500 psi at a temperature within the range of about 200°C to about 320°C for a period of about 1 to about 30 minutes. The particle size of the cellulosic material is then substantially reduced and the internal structure of the cellulosic material is disrupted, by subjecting the pressurized cellulosic material to a substantially reduced second pressure, such as atmospheric pressure. The degree to which the particle size is reduced is dependent upon the pressure conditions and residency time; the degree of particle size reduction of certain pressure and time conditions is set forth in Table I below.

In a presently most preferred embodiment, the cellulosic material is pressurized with steam to a first pressure within the range of about 600 psi to about 800 psi at a temperature within the range of about 250°C to about 270°C for a period of about 1 to about 15 minutes before being subjected to atmospheric pressure. Such a particle size-reducing process is disclosed, for example, in copending application United States Serial No. 195,326 filed October 20, 1980 entitled "Alcohol Manufacturing Process," which is incorporated herein by reference.

A small-scale model of the particle size-reducing process of the above-referenced patent application was conducted by pressurizing cellulosic material (in this case, sawdust) with steam to pressures of about 400-800 psi, for about 1-10 minutes, and subsequently subjecting the cellulosic material to atmospheric pressure. The particle size distributions of the resultant products are given in Table I below. (For purposes of comparison, the particle size distribution for the untreated sawdust is also given in Table I.)

## TABLE I

Experimental Data Obtained From A Small-Scale Model of the Process Disclosed
in United States Patent Application Serial No 195,326 Filed October 20, 1980

|  |  |  | Particle Size Distribution (% of Total Sample) | | | | | |
|---|---|---|---|---|---|---|---|---|
| Pressure (psig) | Temp. (°C) | Time (Min.) | Under 20 Mesh | 20-40 Mesh | 40-80 Mesh | 80-100 Mesh | 100-200 Mesh | Over 200 Mesh |
| 0 Ambient (Untreated Sawdust) | | 0 | 28 | 26 | 33 | 3.2 | 7.6 | 1.7 |
| 400 | 229.4 | 2 | 10 | 27 | 45 | 3 | 7 | 8 |
| 400 | 229.4 | 5 | 8 | 33 | 28 | 2 | 21 | 8 |
| 400 | 229.4 | 10 | 1 | 6 | 32 | 2 | 31 | 28 |
| 500 | 241.7 | 2 | 5 | 16 | 36 | 8 | 17 | 19 |
| 500 | 241.7 | 5 | 4 | 2 | 20 | 2 | 30 | 46 |
| 500 | 241.7 | 10 | 0.1 | 0.9 | 8 | 2 | 18 | 71 |
| 600 | 252.2 | 1 | 1 | 11 | 45 | 6 | 22 | 14 |
| 600 | 252.2 | 4 | 0.0 | 1 | 4 | 1 | 16 | 78 |
| 600 | 252.2 | 8 | 0.5 | 1 | 5 | 1 | 10 | 83 |
| 700 | 261.7 | 1 | 0.3 | 4 | 21 | 3 | 32 | 40 |
| 700 | 261.7 | 4 | 0.6 | 2 | 8 | 0.5 | 14 | 75 |
| 700 | 261.7 | 8 | 0.1 | 0.6 | 3 | 1 | 4 | 92 |
| 800 | 270 | 1 | 0.6 | 2 | 11 | 6 | 23 | 57 |
| 800 | 270 | 4 | 0.5 | 2 | 9 | 2 | 20 | 67 |
| 800 | 270 | 8 | 0 | 0.1 | 1 | 0.3 | 0.4 | 98 |

-23-

It will be recognized that other means for compacting the cellulosic material in accordance with the present invention may be possible. For example, other restriction devices besides restriction grates 28 and 34 could be placed within the reaction vessel so as to restrict the flow of the cellulosic material within the reaction vessel, thereby resulting in compaction of the cellulosic material.

It should also be recognized that one or more of the foregoing embodiments for compacting the cellulosic material in accordance with the present invention may be used either alone or in combination in order to provide the necessary compaction density of the cellulosic material within the reaction vessel.

An important parameter to control in the present invention, is thus the compaction density of the cellulosic material. In the novel apparatus and method of the present invention, it has been found that, in order to obtain the favorable results discussed herein, it is necessary to control the density of the bed of cellulosic material within the reaction vessel such that the density of the bed is within the range of about ten to about thirty pounds per cubic foot (10-30 lbs/ft$^3$).

As mentioned previously herein, minimization of the liquid residence time may be accomplished not only by compaction, but also by vapor displacement of the liquid

-24-

volume within the reaction vessel. Vapor displacement of the liquid volume within the reaction vessel decreases the effective liquid volume within the vessel so as to reduce the liquid residence time, thus having similar effects as compaction of the cellulosic material. Such vapor displacement may be accomplished in many ways, including: (1) sparging an inert gas (such as carbon dioxide) through the liquid within the reaction vessel, and (2) by flashing off a portion of the hot liquid within the reaction vessel.

In one presently preferred embodiment, carbon dioxide gas is sparged into the reaction vessel 24 through acid inlet 30 or acid outlet 32, at a vapor-to-liquid volumetric ratio within the range of about 0.1:1 to about 20:1. The carbon dioxide gas recovered with the sugar solution from outlet 26 may, if desired, be recycled back to inlet 30 or outlet 32.

Where vapor displacement is achieved by flashing off a portion of the hot acid solution (which contains the sugar solution) within the reaction vessel, flashing is accomplished by controlling the pressure within the reaction vessel. In this embodiment, the hot aqueous acid solution within the reaction vessel 24 is exposed to a pressure which drops below the flash point of the acid solution so that bubbles begin to form within the reaction vessel. In the presently preferred embodiment of Figure 1, such flashing is achieved by adjusting valves 27 and 37 such that the pressure within the reaction vessel drops sufficiently below the flash point of the hot acid solution to create a temperature drop in the acid solution of about 0.5°C to about 20°C. Such flashing results in a vapor/liquid flow volumetric ratio within the reaction vessel of about 0.1:1 to about 10:1.

The vapor displacement techniques of the present invention have been found to be most useful in cellulosic beds of a relatively low density, because high density beds have little free liquid available for vapor displacement. In high density beds, the majority of the liquid within the reaction vessel is held by the capillary action of the cellulosic material. In low density beds, such capillary action is not as strong, and the liquid may thus be more easily displaced by vapor.

Again, it should be emphasized that, in the present invention, the liquid residence time may be reduced either by compaction of the bed of cellulosic material, or by vapor displacement of the liquid volume within the reaction vessel, or both.

Other preferable reaction conditions for achieving the desirable results discussed herein in accordance with the present invention are generally as follows, although the exact preferred reaction conditions will depend upon such variables as the nature of the cellulosic feedstock material treated. The temperature within the reaction vessel should preferably be maintained at a temperature within the range of about 170°C to about 260°C, with the presently preferred range being from about 220°C to about 240°C.

The acid to be used may be any suitable hydrolyzing acid, with sulfuric acid being the presently preferred hydrolyzing acid. The concentration of the acid should be from about .01% to about 10% acid by weight, with the presently preferred range being from about 0.05% to about 0.32% acid by weight.

Under these conditions, the values of $k_1$ for reaction (1) are from about 0.04 to about 3, with the preferred range being from about 0.1 to about 2. The corresponding values of $k_2$ for reaction (2) under these reaction conditions are from about 0.05 to about 1, with $k_2$ values of about 0.07 to about 0.75 under the presently preferred reaction conditions disclosed herein.

Also under these reaction conditions, the half life for the hydrolysis reaction is from about 17.3 minutes to about 0.23 minutes (from about 6.9 minutes to about 0.35 minutes under the presently preferred reaction conditions), while the half life for the degradation reaction is from about 13.9 minutes to about 0.69 minutes (from about 9.9 minutes to about 0.92 minutes under the presently preferred reaction conditions). Moreover, at these favorable reaction conditions, smaller reactors than those presently used in the art may be employed with the novel apparatus and method of the present invention.

It will be appreciated that the exact volume of the reaction vessel of the present invention will depend upon the desired feed rate for the solid cellulosic material, the density of the bed of cellulosic material within the reaction vessel, and the value of $k_1$. By way of example, if it were desirable to process one dry ton of sawdust per hour, and if a compaction density of 10 lbs/ft$^3$ were maintained in the cellulosic bed and a $k_1$ value of 0.04 were used, a reaction vessel having a volume of about 151 cubic feet (ft$^3$) would be needed. If the bed density were increased to 20 lbs/ft$^3$ and the $k_1$ value were increased to 3.0, a reaction vessel volume of about 0.81 ft$^3$ would be required to process the same amount of sawdust.

The solid/liquid flow ratio maintained within the reaction vessel is primarily determined by the desired concentration of fermentable sugars in the acid solution removed from the reaction vessel. By way of example, if it were desirable to achieve a 14% fermentable sugar concentration in the acid solution (prior to flash cooling), the solid/liquid flow ratio would be about 1:2.8, or about 0.35 pounds per minute (lbs/min) of sawdust for each pound per minute of acid. To achieve an 8% fermentable sugar concentration in the acid solution, a solid/liquid flow ratio of about 1:5.7 or about 0.18 lbs/min of sawdust for each pound per minute of acid would be required.

In order to predict the benefits of compaction of the cellulosic bed and vapor displacement of the liquid volume within the reaction vessel, a computer model of the countercurrent acid hydrolysis process of the present invention has been developed. According to this model, the predicted recoveries of the total available sugar were calculated for a sugar concentration of about 8.8%. The predicted sugar recoveries for various compaction densities using different reaction vessel volumes are given in Table I below.

Table I

| Compaction Density ($lbs/ft^3$) | Reactor Volume ($ft^3$) | Glucose Recovery(%) | Reducing Sugar Recovery(%) | Concentration of Reducing Sugar (wt.%) |
|---|---|---|---|---|
| 10 | 2.88 | 69.7 | 69.6 | 8.8 |
| 15 | 1.92 | 81.2 | 81.2 | 8.8 |
| 20 | 1.44 | 86.8 | 86.7 | 8.8 |
| 25 | 1.15 | 90.1 | 90.1 | 8.8 |

It is noteworthy that the percent recovery of sugars was from about 70-90% using a compaction density within the

-29-

cellulosic bed of 10-25 pounds per cubic foot in accordance with the present invention.

A computerized model of the effects of vapor displacement within the reaction vessel having a compaction density of 10 lbs/ft$^3$ was also generated. The results of this computerized model are reported in Table II below.

### Table II

| Percent of Reactor Volume Displaced by Vapor | Reactor Volume (ft$^3$) | Glucose Recovery(%) | Reducing Sugar Recovery(%) | Concentration of Reducing Sugar (wt.%) |
|---|---|---|---|---|
| 0% | 2.88 | 69.7 | 69.6 | 8.8 |
| 10% | 2.88 | 72.6 | 72.3 | 8.8 |
| 15% | 2.88 | 73.7 | 73.7 | 8.9 |
| 20% | 2.88 | 75.5 | 75.4 | 8.8 |

As can be seen from Table II above, an increase in the recovery of sugars from a little less than 70% to a little more than 75% was predicted for this computerized model when 20% of the reactor volume was displaced by vapor in accordance with the present invention.

From the foregoing, it will be appreciated that the method and apparatus of the present invention may be employed in an alcohol manufacturing process. In such a process, virtually any cellulosic starting material, _e.g._,

corn residue, forestry, feed lot, crop, urban, or industrial wastes, may be employed. The cellulosic starting material is comminuted, if necessary, and the material is treated in accordance with the method and apparatus of the present invention for continuously hydrolyzing cellulosic material. The sugar solution recovered from the hydrolysis process may then be converted to alcohol by any suitable process, such as the process described hereinbelow.

The sugar solution is first neutralized, typically to a pH of about 4.5-6.0, with a base such as sodium hydroxide, calcium hydroxide, or any other suitable base. This slightly acidic slurry is typically referred to as the "wort." Yeast nutrients are added to the wort as the wort cools to a temperature suitable for fermentation, typically about 30-32°C.

Once cooled, the wort is introduced into a fermentation vessel containing yeast and the yeast feeds on the sugars in the wort to produce ethanol and carbon dioxide as byproducts. The carbon dioxide is removed as a gas, while the ethanol stays in solution. In typical fermentation processes, ethanol and carbon dioxide are produced in relative percentages of about 51% and 49%, respectively.

Once fermentation is complete, the ethanol-containing spent wort is sent to a distillation column for separation of the ethanol. Ethanol, which has a lower boiling point

-31-

than water, is separated from the spent wort in the distillation column, and may be purified to the degree desired.

In view of the foregoing, it will be appreciated that the present invention provides an improved countercurrent flow apparatus and method for continuously hydrolyzing cellulosic material to sugars, which sugars can be used, for example, in the production of alcohols such as ethanol. Furthermore, the present invention provides an apparatus and method for continuously hydrolyzing cellulosic material wherein a maximum amount of the available sugar content of the cellulosic material is recovered, while maintaining a high concentration of sugar in the sugar solution recovered and while minimizing degradation of the sugars produced.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

What is claimed and desired to be secured by Letters Patent is:

CLAIMS

1. A continuous method, comprising the steps of:

continuously introducing cellulosic material into a first end of a reaction vessel, said cellulosic material forming a bed within the reaction vessel;

continuously introducing an acid solution into a second end of the reaction vessel so as to contact the cellulosic material in a countercurrent fashion such that the acid solution hydrolyzes the cellulosic material to produce a sugar solution and lignin;

controlling the density of the bed of cellulosic material such that the density is within the range of about 10 to about 30 pounds per cubic foot, and/or controlling the volume of the acid solution within the reaction vessel by displacing a portion of the acid solution with a gas or vapour;

removing the sugar solution from the first end of the reaction vessel; and

removing the lignin from the second end of the reaction vessel.

2. A continuous method as defined in claim 1 wherein the density of the bed is controlled by reducing the particle size of at least a portion of the cellulosic material prior to introduction of the cellulosic material into the reaction vessel.

3. A continuous method as defined in claim 2 wherein the particle size of at least a portion of the cellulosic material is reduced by a steam disruption process comprising the steps of:

pressurizing the cellulosic material with steam to a first pressure within the range of about 250 psi to about 1500 psi for a period of about 1 to about 30 minutes; and

disrupting the structure of at least a portion of the cellulosic material by subjecting the cellulosic material to a substantially reduced second pressure.

4. A continuous method as defined in claim 3 wherein the second pressure is about atmospheric pressure.

5. A continuous method as defined in claim 3 or 4 wherein the cellulosic material is pressurized with steam to a first pressure within the range of about 600 psi to about 800 psi for a period of about 1 to about 15 minutes.

6. A continuous method as defined in any of claims 1 to 5 wherein the density of the bed is controlled by compacting the cellulosic material which forms the bed.

7. A continuous method as defined in claim 6 wherein the cellulosic material is compacted by a grate mounted within the reaction vessel.

8. A continuous method as defined in any of claims 1 to 7 wherein the volume controlling step comprises sparging gas into the acid solution so as to displace a portion of the acid solution within the reaction vessel.

9. A continuous method as defined in claim 8 wherein the gas sparged into the acid solution comprises carbon dioxide.

10. A continuous method as defined in any of claims 1 to 7 wherein the volume controlling step comprises flashing off a portion of the acid solution to form a vapour and thereby displace a portion of the acid solution within the reaction vessel.

11. A continuous method as defined in any of claims 1 to 10 wherein the volumetric ratio of the gas or vapour to the acid solution is from about 0.1:1 to about 20:1.

12. A continuous method as defined in any of claims 1 to 11 wherein the temperature within the reaction vessel is within the range of about $170^{\circ}C$ to about $260^{\circ}C$.

13. A continuous method as defined in any of claims 1 to 12 wherein the acid solution is in a concentration of about .01% to about 10% acid by weight.

14. A continuous method as defined in any of claims 1 to 13 wherein the acid of the acid solution is sulfuric acid.

15. A continuous method as defined in any of claims 1 to 14 further comprising the steps of:

placing the sugar solution into a fermentation vessel;

mixing yeast with the sugar solution and fermenting the sugar solution to produce alcohol; and

distilling the alcohol in a distillation column so as to purify the alcohol.

16. An apparatus comprising:

a reaction vessel;

first inlet means disposed at a first end of said reaction vessel for receiving cellulosic material into said reaction vessel;

second inlet means disposed at a second end of said reaction vessel for receiving an acid solution into said reaction vessel;

means for compacting the cellulosic material to a density within the range of about 10 to about 30 pounds per cubic foot, and/or means for displacing a portion of said acid solution within the reaction vessel so as to control the volume of the acid solution.

first outlet means disposed at said first end of said reaction vessel for allowing removal of sugar solution from said reaction vessel; and

second outlet means disposed at said second end of said reaction vessel for allowing removal of lignin from said reaction vessel.

17. An apparatus as defined in claim 16 wherein said compacting means comprises a grate mounted within said reaction vessel between said first inlet means and said second outlet means.

18. An apparatus as defined in claim 19 further comprising a second grate mounted within said reaction vessel for further compacting the cellulosic material.

19. An apparatus as defined in claim 16 wherein said compacting means comprises means for reducing the particle size of at least a portion of the cellulosic material prior to introduction of the cellulosic material into the reaction vessel.

20. An apparatus as defined in any of claims 16 to 19 wherein said displacing means comprises means for sparging gas into said reaction vessel.

21. An apparatus as defined in any of claims 16 to 19 wherein said displacing means comprises means for flashing off a portion of said acid solution within said reaction vessel to form a vapor within said reaction vessel.

22. An apparatus as defined in claim 21 further comprising means for controlling the pressure within said reaction vessel so as to control the degree of flashing of said acid solution.

23. An apparatus as defined in any of claims 16 to 22 further comprising:

a fermentation vessel for receiving said sugar solution from said first outlet means of said reaction vessel, said sugar solution being fermented within said fermentation vessel to produce alcohol; and

a distillation column for purifying said alcohol from said fermentation vessel.

0134647

1/1

Fig. 1

Fig. 2

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| Y | FR-A- 706 678 (HEINRICH SCHOLLER) <br> * Page 1, lines 45-50; page 2, lines 36-44; summary; figure 1 * | 1-23 | C 13 K 1/02 |
| Y | FR-A- 933 025 (EMPRESA NACIONAL CALVO SOTELO) <br> * Summary; page 2, lines 5-10 * | 1-23 | |
| Y | CHEMICAL ABSTRACTS, vol. 97, no. 24, 13th December 1982, page 91, no. 199738c, Columbus, Ohio, USA; S.K. SONG et al.: "Countercurrent reactor in acid catalyzed cellulose hydrolysis" & CHEM. ENG. COMMUN. 1982, 17(1-6), 23-30 <br> * Abstract * | 1-23 | |
| D,A | US-A-2 681 871 (LEWIS C. WALLACE) | | TECHNICAL FIELDS SEARCHED (Int. Cl.4) <br><br> C 13 K |

The present search report has been drawn up for all claims

| Place of search | Date of completion of the search | Examiner |
|---|---|---|
| THE HAGUE | 31-10-1984 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82